# EUROPÄISCHE PATENTSCHRIFT

(11) **EP 3 334 372 B1**
(45) Veröffentlichungstag und Bekanntmachung des Hinweises auf die Patenterteilung: **01.05.2024**
(21) Anmeldenummer: 16748319.7
(22) Anmeldetag: 10.08.2016
(51) Int. Cl.: A61C 13/00

(54) **VERFAHREN ZUM HERSTELLEN EINES ANATOMISCHEN ZAHNIMPLANTATS**
METHOD FOR PRODUCING AN ANATOMICAL DENTAL IMPLANT
PROCÉDÉ DE FABRICATION D'UN IMPLANT DENTAIRE ANATOMIQUE

(30) Priorität: 14.08.2015 DE 102015215587
(43) Veröffentlichungstag der Anmeldung: 20.06.2018
(73) Patentinhaber: nt-trading GmbH & Co. KG, 76187 Karlsruhe (DE)
(72) Erfinder: JAHN, Dirk, 76835 Weyher (DE)
(74) Vertreter: Patentanwälte Ruff, Wilhelm, Beier, Dauster & Partner mbB
(86) Internationale Anmeldenummer: PCT/EP2016/069054
(87) Internationale Veröffentlichungsnummer: WO 2017/029168

(56) Entgegenhaltungen:
- WO-A1-2013/096592
- CN-U- 202 724 012
- US-A1- 2009 325 128
- US-A1- 2010 203 478
- US-A1- 2012 251 980

## Beschreibung

Die Erfindung betrifft ein Verfahren zum Herstellen eines Knochenersatzstücks.

Knochenersatzstücke können in menschliche oder tierische Knochen eingefügt werden, um ein nicht mehr vorhandenes oder zu entfernendes Knochenstück zu ersetzen. Beispielsweise kann ein solcher Ersatz notwendig werden, wenn eine Zahnwurzel krankheitsbedingt zerstört, nicht veranlagt oder anderweitig defekt ist, oder wenn aufgrund eines Unfalls ein Teil eines Knochens herausgeschlagen wurde.

Bei Knochenersatzstücken ist es im Regelfall notwendig, eine möglichst gute Passung zwischen Knochenersatzstück und einer Kavität, in welcher das Knochenersatzstück aufgenommen werden soll, herzustellen. Dies ermöglicht eine spielfreie Passung und einen langfristig guten Sitz.

Bekannte Verfahren zum Herstellen eines Knochenersatzstücks basieren typischerweise darauf, dass mittels einer Masse, welche sich in der Kavität verfestigen kann, ein Abdruck der Kavität genommen wird und basierend darauf das Knochenersatzstück hergestellt wird. Dies bringt allerdings den Nachteil mit sich, dass hierzu ein Eingriff am Patienten vorgenommen werden muss, und dass überdies das Ausbilden eines solchen Abdrucks erst dann möglich ist, wenn die Kavität frei und von außen zugänglich ist. Das Herstellen eines Ersatzstücks für eine Zahnwurzel ist beispielsweise somit erst möglich, wenn die Zahnwurzel aus dem Kiefer entfernt wurde. Dies kann aufgrund der damit verbundenen Zeitdauer eine erhebliche Belastung für den Patienten darstellen. Außerdem erzeugen gerade manuell durchzuführende Schritte Fehlerquellen, welche zu Fehlern bei der Behandlung des Patienten führen können.

Künstlicher Zahnersatz und Teile davon sowie Verfahren zur Herstellung von künstlichem Zahnersatz bzw. Teilen davon sind aus der US 2009/0325128 A1, US 2010/0203478 A1, CN 202 724 012 U, WO 2013/096592 A1 sowie US 2012/0251980 A1 bekannt.

### Aufgabe und Lösung

Es ist deshalb eine Aufgabe der Erfindung, ein Verfahren zum Herstellen eines Knochenersatzstücks bereitzustellen, welches beispielsweise eine schnellere oder weniger Eingriffe am Patienten erfordernde Durchführung erlaubt.

Dies wird erfindungsgemäß durch ein Verfahren nach Anspruch 1 erreicht. Vorteilhafte Ausgestaltungen können beispielsweise den abhängigen Ansprüchen entnommen werden. Der Inhalt der Ansprüche wird durch ausdrückliche Inbezugnahme zum Inhalt der Beschreibung gemacht.

Die Erfindung betrifft ein Verfahren zum Herstellen eines Knochenersatzstücks gemäß dem unabhängigen Anspruch 1. Bevorzugte Ausführungsformen sind in den abhängigen Ansprüchen definiert.

Mittels des erfindungsgemäßen Verfahrens ist eine weitgehende Automatisierung der Herstellung eines Knochenersatzstücks möglich. Dies kann nötige Eingriffe und die insgesamt erforderliche Zeit deutlich verringern, was eine erhebliche Erleichterung für den Patienten darstellt. Insbesondere kann der Ablauf der Herstellung des Knochenersatzstücks weiter automatisiert werden, so dass auch das Risiko eventueller menschlicher Fehler minimiert wird.

Bei einer Kavität kann es sich typischerweise um einen Hohlraum, insbesondere um einen von außen zugänglichen Hohlraum, in einem menschlichen oder tierischen Knochen handeln. Dieser kann entweder absichtlich, beispielsweise mittels medizinischer Instrumente oder durch Herausziehen eines Zahns, oder auch unabsichtlich wie beispielsweise bei einem Unfall erzeugt werden. Die Kavität soll im Rahmen des Verfahrens typischerweise wieder gefüllt werden, so dass im Idealfall der Patient nach seiner Behandlung nicht mehr merkt, dass die Kavität ursprünglich einmal ungefüllt im Körper vorhanden war.

Bei der Struktur der Kavität kann es sich insbesondere um die dreidimensionale Struktur der Kavität handeln. Diese kann in geeigneter Weise durch die Ausgangsdaten beschrieben werden. Beispielsweise kann die Kavität in dreidimensionale Pixeldaten umgesetzt werden. Die Kavität kann beispielsweise auch als Vektordatei beschrieben werden.

Die Bearbeitungsdaten sind typischerweise auf einer anderen Ebene angesiedelt als die Ausgangsdaten. Während die Ausgangsdaten typischerweise auf einer Messung basieren, sind die Bearbeitungsdaten dazu ausgelegt, dass sie typischerweise unmittelbar für die automatisierte Herstellung des Knochenersatzstücks verwendet werden können. Beispielsweise kann es sich dabei um NC- oder um CNC-Daten handeln, welche unmittelbar in einer Werkzeugmaschine zur automatisierten Herstellung des Knochenersatzstücks verwendet werden können.

Das Knochenersatzstück ist ein Zahnimplantat, ein Abutment und/oder eine Zahnprothese (künstliche Zahnkrone) .

Unter dem Ausdruck "Abutment" soll im Sinne der vorliegenden Erfindung eine Verbindungsstruktur bzw. ein Verbindungsteil (Mesostruktur) zwischen einem Zahnimplantat und einer Zahnprothese verstanden werden. Bei einem konfektionierten Implantat dient das Abutment insbesondere dazu, insertionsbedingte Abwinklungen des Implantats auszugleichen, um in einer idealen Position eines nicht mehr vorhandenen Zahns die prothetische Versorgung aufnehmen zu können. Die Verbindung zum Implantat erfolgt vorzugsweise durch eine Verschraubung, die einen Schraubenkanal und eine gewisse Steifigkeit bedingt. Das Abutment kann beispielsweise auch federnde und/oder haftende Komponenten enthalten.

Bei dem Knochenersatzstück kann es sich insbesondere um ein Zahnimplantat mit oder auch ohne Abutment-Struktur handeln. Dabei können das Zahnimplantat und die Abutment-Struktur ein einstückig ausgebildetes Knochenersatzstück darstellen.

Die Kavität kann insbesondere in einem Unterkiefer oder in einem Oberkiefer zur Aufnahme einer Zahnwurzel angeordnet sein. Eine solche Kavität ist typischerweise im gesunden Zustand mit einer Zahnwurzel gefüllt. Wird der Zahn gezogen, so ist eine solche Kavität typischerweise leer und wird durch Einsetzen eines Zahnimplantats wieder gefüllt. Krankheits- oder unfallbedingt oder aus prothetischen Gründen kann sich die Kavität auch über den Bereich einer Zahnwurzel hinaus erstrecken.

Gemäß einer bevorzugten Ausführung wird der Schritt des Vermessens der Kavität durchgeführt, während sich eine Zahnwurzel oder Zahnwurzelersatz und/oder ein Zahn oder Zahnersatz in der Kavität befinden. Dies ermöglicht eine besonders geringe Belastung für den Patienten, da der Zahn erst dann gezogen werden muss, wenn das Knochenersatzstück bereits fertig hergestellt wurde. Der Patient muss also nicht tagelang mit einem gezogenen Zahn bzw. einer dadurch entstandenen Zahnlücke herumlaufen, um auf die Fertigstellung des Zahnimplantats zu warten.

Der Schritt des Vermessens der Kavität wird bevorzugt mittels Computertomographie (CT), Magnetresonanztomographie (MRT), digitaler Volumentomographie (DVT) oder 3D-Scan durchgeführt. Derartige Verfahren haben sich für die Durchführung der Erfindung als vorteilhaft erwiesen. Insbesondere ermöglichen sie das exakte Erfassen und Auswerten einer Kavität in einem Knochen, ohne dass hierzu Füllmaterial oder sonstiges härtendes Material in die Kavität eingebracht werden muss. Der Patient legt sich für derartige Verfahren typischerweise auf eine Liege bzw. sitzt oder steht in einer bestimmten Position, wobei die Kavität weitgehend automatisch erfasst wird. Außerdem können Fehler aufgrund von Luftblasen, hängengebliebenem Abformmaterial oder sonstigen Fehlerquellen zuverlässig ausgeschlossen werden.

Bevorzugt werden die Ausgangsdaten in Computer-Aided-Design (CAD)-Daten konvertiert, wobei basierend auf den CAD-Daten die Bearbeitungsdaten erzeugt werden. Derartige CAD-Daten können insbesondere Vektordaten sein. Solche CAD-Daten ermöglichen die Umsetzung der Ausgangsdaten in gut weiterverarbeitbare und bei Bedarf auch manuell bearbeitbare Daten mit definierter Struktur und definierter Auflösung.

Die Bearbeitungsdaten werden bevorzugt abhängig von der Kavität und/oder einer Position der Kavität erzeugt. Dies ermöglicht die Berücksichtigung der Art der Kavität oder einer Position der Kavität. Beispielsweise kann bei der Erstellung der Bearbeitungsdaten berücksichtigt werden, ob sich die Kavität in einem Oberkiefer, in einem Unterkiefer, an welcher Stelle im jeweiligen Kiefer oder auch in einem völlig anderen Knochen eines menschlichen oder tierischen Körpers befindet. Abhängig davon können unterschiedliche Parameter bei der Erzeugung der Bearbeitungsdaten verwendet werden, welche den jeweiligen lokalen Gegebenheiten wie beispielsweise Verformbarkeit des Knochens oder Belastbarkeit der jeweiligen Struktur Rechnung tragen.

Bevorzugt wird auf die Bearbeitungsdaten vor dem Herstellen des Knochenersatzstücks eine Finite-Elemente-Analyse angewendet. Insbesondere erfolgt dies unter Berücksichtigung von Daten bezüglich Oberkiefer, Unterkiefer, Okklusion und/oder Knochenstruktur. Mittels einer solchen Finite-Elemente-Analyse können die Daten aufbereitet und besser für ein bestimmtes Gerät zum Herstellen des Knochenersatzstücks angepasst werden. Die Finite-Elemente-Analyse unterstützt mit besonderem Vorteil die Analyse biomechanischer Systeme, wie beispielsweise von Knochen, Sehnen, Bändern und sogar von Blutgefäßen. Durch die Finite-Elemente-Analyse als berührungsloses Verfahren sind insbesondere Messungen mit einem wesentlichen größeren Dynamikbereich möglich als mit herkömmlichen Messmethoden.

Unter einer Okklusion soll im Sinne der vorliegenden Erfindung insbesondere die statische und dynamische Kontaktbeziehung zwischen Oberkieferzähnen und Unterkieferzähnen verstanden werden. Diese Kontaktbeziehung muss harmonisch/funktionell ausgebildet sein, um eine Schädigung des stomatognathen Systems zu vermeiden. Dental findet die Okklusion im Bereich der Zahn-Kauflächen und dem entsprechenden Antagonist statt. Im weitesten Sinn kann die Kaufläche, welche geprägt wird durch Höcker, Abhänge und Fissuren, als eine gefüllte Kavität bezeichnet werden.

Gemäß einer bevorzugten Ausführung weist das Verfahren ferner den Schritt eines Optimierens und/oder Re-Designens (erneutes Erzeugen) der Bearbeitungsdaten vor dem Herstellen des Knochenersatzstücks auf. Damit kann die Struktur des Knochenersatzstücks optimiert werden. Das Optimieren und/oder das Re-Designen können jeweils sowohl manuell wie auch automatisiert, beispielsweise mit einem fest implementierten Algorithmus durchgeführt werden. Bei einem Re-Design können insbesondere möglicherweise auftretende Spannungen ausgeglichen werden.

Das Herstellen des Knochenersatzstücks kann durch Fräsen und/oder ein generatives Fertigungsverfahren, wie beispielsweise 3D-Druck, erfolgen.

Gemäß einer weiteren Ausführung weist das Knochenersatzstück ein Material auf oder ist aus einem Material gebildet, welches ausgewählt ist aus der Gruppe aufweisend Metalle, Polymere, synthetische Polymere, Biopolymere (natürlich vorkommende Polymere), Keramiken, Zementmaterialien und Kombinationen, insbesondere Mischungen oder Komposite, davon.

Beispielsweise kann das Knochenersatzstück ein Material aufweisen oder aus einem Material gebildet sein, welches ausgewählt ist aus der Gruppe aufweisend Titan, Proteine, Gelatine, Kollagen, Polysaccharide, Mucopolysaccharide, Alignat, Hyaluronsäure, Polyetherketon, Polyetheretherketon, Phosphate, Calciumphosphate, Octacalciumphosphat (OCP), Apatit, Hydroxylapatit, Phosphatkeramiken, Calciumphosphatkeramiken, Apatitkeramiken, Hydrooxylapatitkeramiken, und Kombinationen, insbesondere Mischungen oder Komposite, davon.

Das Knochenersatzstück kann insbesondere Octacalciumphosphat (OCP) und Biopolymere, wie beispielsweise Gelatine, Kollagen, Alginat und/oder Hyaluronsäure, aufweisen oder aus diesen Materalien bestehen.

Gemäß einer weiteren Ausführung ist das Knochenersatzstück als Titanschaum, insbesondere poröser Titanschaum, vorzugsweise offenporiger Titanschaum, ausgestaltet.

Insbesondere kann das Knochenersatzstück eine Mikrostruktur, d. h. eine Struktur mit einer Porengröße im µm-Bereich (Mikrometerbereich), aufweisen. Bevorzugt weist die Mikrostruktur eine Porengröße < 2 nm auf. Weiterhin können die Poren der Mikrostruktur wabenförmig ausgestaltet sein.

Vorzugsweise weist das Knochenersatzstück eine mikroporöse Titanstruktur, d. h. eine Titanstruktur mit einer Porengröße in µm-Bereich (Mikrometerbereich), auf. Insbesondere kann das Knochenersatzstück eine Titanstruktur mit einer Porengrößer < 2 nm aufweisen. Weiterhin können die Poren wabenförmig ausgestaltet sein. Erfindungsgemäß kann es somit vorgesehen sein, dass das Knochenersatzstück eine sogenannte Mikro-Waben-Titanstruktur aufweist.

Bevorzugt weist das Verfahren ferner einen Schritt eines Überprüfens des Knochenersatzstücks nach dem Herstellen mittels Computertomographie (CT), Magnetresonanztomographie (MRT) oder digitaler Videotomographie (DVT) auf. Damit kann überprüft werden, ob das Knochenersatzstück richtig hergestellt wurde, bevor es einem Arzt geliefert wird bzw. in einen Patienten eingesetzt wird. Unnötige Behandlungen mit fehlerhaften Knochenersatzstücken und die damit verbundene Belastung für den Patienten sowie das Risiko weiterer Schäden können auf diese Weise vorteilhaft vermieden werden. Es sei erwähnt, dass auch andere Vorgehensweisen als die eben erwähnten zum Überprüfen des Knochenersatzstücks verwendet werden können. Insbesondere kann die gleiche Vorgehensweise zur Überprüfung des Knochenersatzstücks verwendet werden, welche auch zum Vermessen der Kavität verwendet wird. Dies kann apparativen Aufwand einsparen.

Bevorzugt weist das Verfahren ferner einen Schritt des Nachbehandelns des Knochenersatzstücks nach dem Herstellen auf, und zwar insbesondere in Abhängigkeit von einer Überprüfung, und insbesondere an einem Kieferknochenbereich, Zahnwurzelbereich oder an einem Zahnfleischkontaktbereich. Das Nachbehandeln kann beispielsweise in Abhängigkeit oder ansprechend auf das Überprüfen des Knochenersatzstücks erfolgen. Beispielsweise können erkannte Fehler in dem Knochenersatzstück korrigiert werden, insbesondere durch Abtragen von überflüssigem Material oder durch Hinzufügen von fehlendem Material. Dies erlaubt eine noch bessere Passung des Knochenersatzstücks an die Kavität, selbst bei Bearbeitungsfehlern, welche im Rahmen des Herstellungsprozesses auftreten können.

Das Knochenersatzstück ist ein Zahnimplantat. Dies entspricht einer typischen und häufigen Anwendung, da Zähne häufig aufgrund diverser Schäden ganz oder teilweise ersetzt werden müssen. Außerdem ist das Verfahren in diesem Fall von besonderem Vorteil, da die Zeit, welche die Herstellung des Knochenersatzstücks benötigt und welche ein Patient gegebenenfalls mit einer Zahnlücke oder einem Provisorium leben muss, minimiert werden kann.

Bevorzugt wird beim Schritt des Vermessens der Kavität auch ein in der Kavität befindlicher Zahn oder Zahnersatz vermessen, und zwar insbesondere zur Erzeugung von weiteren Ausgangsdaten, welche anzeigend sind für eine Struktur, insbesondere eine Oberfläche, des Zahns. Dies ermöglicht eine Integration der Herstellung einer Zahnprothese in den Verfahrensablauf. Auf zusätzliche Schritte oder auf die Verwendung separater Geräte kann vorteilhaft verzichtet werden.

Es sei verstanden, dass ein Zahn auch unabhängig von der Kavität vermessen werden kann und die hieraus gewonnen Daten beispielsweise zur Herstellung einer Zahnprothese verwendet werden können. Hierbei kann auf die anderen hierin beschriebenen Ausführungen und Varianten entsprechend zurückgegriffen werden.

Weiter bevorzugt werden basierend auf den weiteren Ausgangsdaten weitere Bearbeitungsdaten erzeugt, welche anzeigend sind für eine Soll-Struktur der Zahnprothese und/oder eine Soll-Struktur der Prothesenkronen-Brücken-Absorptionskomponente. Derartige Bearbeitungsdaten können in ähnlicher Weise wie die bereits weiter oben angesprochenen Bearbeitungsdaten verwendet werden, um eine automatisierte Herstellung zu ermöglichen, wobei in diesem Fall wie erwähnt die Zahnprothese und/oder die Prothesenkronen-Brücken-Absorptionskomponente hergestellt werden. Unter einer Prothesenkronen-Brücken-Absorptionskomponente wird dabei insbesondere ein Element verstanden, welches zwischen einer Zahnprothese und einem Zahnimplantat angeordnet ist und dazu ausgebildet ist, Stöße oder andere Krafteinwirkungen abzufedern. Die weiteren Ausgangsdaten, welche anzeigend sind für die Sollstruktur der Zahnprothese, können vorteilhaft mittels Computer-Aided-Design (CAD) erzeugt werden. Zu den damit erreichbaren Vorteilen sei auf die weiter oben gegebenen Ausführungen verwiesen.

Bevorzugt weist das Verfahren ferner einen Schritt des Herstellens der Zahnprothese basierend auf den weiteren Ausgangsdaten auf, wobei das Herstellen insbesondere durch Fräsen und/oder ein generatives Fertigungsverfahren, wie beispielsweise 3D-Druck, erfolgen kann. Dies ermöglicht eine besonders vorteilhafte Integration der Herstellung des Zahnimplantats zusammen mit der Zahnprothese, wobei insgesamt nur eine minimale Anzahl von Bearbeitungsvorgängen erforderlich ist.

Die Zahnprothese kann insbesondere einstückig mit dem Zahnimplantat ausgebildet sein. Dies erlaubt eine einfache Herstellung und eine stabile Struktur. Sie kann jedoch auch separat vom Zahnimplantat ausgebildet sein, was beispielsweise die Verwendung besonderer separater Herstellungstechniken oder das Vorsehen von speziellen Komponenten zwischen Zahnimplantat und Zahnprothese ermöglichen kann.

Das Verfahren weist bevorzugt ferner einen Schritt des Überprüfens der Zahnprothese auf, und zwar insbesondere mittels Computertomographie (CT) oder 3D-Scan. Damit kann in ähnlicher Weise wie weiter oben bereits beschrieben, vor dem Einsetzen der Prothese sichergestellt werden, dass diese korrekt hergestellt wurde. Fehlbehandlungen und die damit verbundenen Komplikationen können vermieden werden.

Weiter bevorzugt weist das Verfahren einen Schritt der Weiterbehandlung der Zahnprothese auf, welcher insbesondere eine Oberflächenbehandlung beinhalten kann. Die Weiterverarbeitung beinhaltet dabei vorzugsweise eine Beschichtung eines Kieferknochen- und/oder Zahnfleischkontaktbereichs, eine Sterilisation und/oder eine Verpackung.

Mittels einer Weiterbehandlung, insbesondere in Form einer Oberflächenbehandlung, kann ein etwaiger Bearbeitungsfehler korrigiert werden, so dass trotz gewisser Unzulänglichkeiten in der Herstellung die gewünschte Zahnprothese erhalten wird bzw. verwendet werden kann. Ein Kieferknochen- und/oder Zahnfleischkontaktbereich kann beispielsweise mit einem porösen Material beschichtet werden, welches eine bessere Verbindung zum Kieferknochen- und/oder Zahnfleisch herstellt. Eine Sterilisation kann der Entfernung bzw. Abtötung von Krankheitserregern dienen. Eine Verpackung kann insbesondere die Prothese für einen Versand zu einem Arzt, beispielsweise auf dem Postweg, vorbereiten.

Das Verfahren weist ferner einen Schritt des Ermittelns von jeweiligen Typen einer Anzahl von chirurgischen Instrumenten auf und zwar basierend auf den Ausgangsdaten, den Bearbeitungsdaten, einem Design (Form) des Zahnimplantats, einem Design (Form) des Abutments und/oder einem Design (Form) der Zahnprothese. Dies ermöglicht die Verwendung der im Rahmen des Verfahrens anfallenden Daten oder auch separater Daten, um einem Arzt die Behandlung insofern zu erleichtern, als unmittelbar notwendige Instrumente wie beispielsweise Zangen oder Bohrer vorausgewählt werden. Der Arzt muss somit nicht mehr vor der Behandlung selbständig überlegen, welche Instrumente er für die Behandlung benötigt. Beispielsweise kann im Fall, dass ein Wurzelkanal vorhanden ist oder bearbeitet werden soll, ein bestimmter Werkzeugsatz ausgewählt werden. Ebenso kann das Design berücksichtigt werden, beispielsweise hinsichtlich Größe oder Oberflächenbeschaffenheit, um hierfür geeignete Werkzeuge zu verwenden.

Bevorzugt weist das Verfahren ferner einen Schritt des Ermittelns von Navigationsinformationen auf, und zwar insbesondere in Bezug auf einen Unterkiefer oder einen Oberkiefer. Dieser Schritt kann insbesondere auf den Ausgangsdaten, den Bearbeitungsdaten, einem Wurzelkanal, einem Design des Zahnimplantats, einem Design des Abutments und/oder einem Design der Zahnprothese basieren. Derartige Navigationsinformationen können vorgefertigte Informationen für den Arzt sein, welche ihm die Behandlung erleichtern, so dass er beispielsweise unmittelbar weiß, an welcher Stelle des Körpers oder eines Kiefers eine Behandlung erfolgen soll. Die Navigationsinformationen können auch so aufbereitet werden, dass sie unmittelbar in elektronischer Form weiterverarbeitet werden können, beispielsweise für ein Augmented-Reality-System. Beispielsweise kann der Arzt eine Brille oder ein head-up-Display verwenden, welche die Einblendung solcher Navigationsinformationen ermöglicht. Dies erlaubt eine Führung und Information des Arztes während der Behandlung, ohne dass er hierfür die Behandlung unterbrechen muss, um Informationen nachzuschlagen.

### Kurze Beschreibung der Zeichnung

Weitere Merkmale und Vorteile wird der Fachmann den nachfolgend mit Bezug auf die beigefügten Zeichnungen beschriebenen Ausführungsbeispielen entnehmen.

Dabei zeigen:
Fig. 1: ein System zum automatisierten Herstellen eines Knochenersatzstücks,
Fig. 2: ein Zahnfleisch mit Kavität und Zahn,
Fig. 3: ein erstes Ausführungsbeispiel eines Knochenersatzstücks,
Fig. 4: ein zweites Ausführungsbeispiel eines Knochenersatzstücks.

### Detaillierte Beschreibung der Ausführungsbeispiele

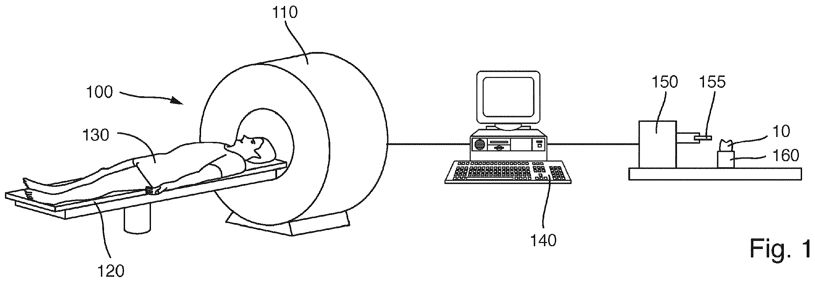

Fig. 1 zeigt ein System 100 zum Herstellen eines Knochenersatzstücks 10.

Das System 100 weist einen Computertomographen (CT) 110 auf. Vor diesem ist eine Liege 120 angeordnet, auf welcher vorliegend ein Patient 130 liegt. Die Liege 120 kann in den Computertomographen 110 eingeschoben werden, so dass der Patient 130 mittels des Computertomographen 110 untersucht werden kann. Dies erlaubt insbesondere die Vermessung einer Kavität in einem Knochen des Patienten 130, für welche ein Knochenersatzstück hergestellt werden soll. Insbesondere kann damit eine Kavität zur Aufnahme eines Zahns und auch der darin befindliche Zahn vermessen werden.

Das System 100 weist ferner einen Computer 140 auf, welcher mit dem Computertomographen 110 zum Empfang von Daten verbunden ist. Der Computertomograph 110 erzeugt abhängig von seiner Messung am Patienten 130 Ausgangsdaten, welche an den Computer 140 geliefert werden. Diese können sowohl anzeigend für eine Kavität in einem Knochen des Patienten 130 sein, wie auch anzeigend für einen zu imitierenden Knochen wie beispielsweise einen Zahn, für welchen eine Prothese hergestellt werden soll. Es sei erwähnt, dass der Computertomograph 110 auch durch den Computer 140 gesteuert werden kann.

Der Computer 140 ist dazu konfiguriert, die Ausgangsdaten in Computer-Aided-Design(CAD)-Daten zu konvertieren. Diese CAD-Daten können einem Benutzer angezeigt werden, so dass dieser eine Überprüfung vornehmen kann, was nachfolgende Bearbeitungsschritte mit falschen Daten verhindert.

Für die CAD-Bearbeitung wird ein sogenanntes 3D-Volumenmodell, d. h. ein virtuelles Modell, des Knochenersatzstücks erzeugt. Das 3D-Volumenmodell wird üblicherweise in einem CAD-Programm als Konstruktionsbasis für das Design eines neuen 3D-Modells verwendet. Allerdings kann das 3D-Modell auch in der vorhandenen Form oder durch Ergänzung von weiteren 3D-Modellen aus Datenbibliotheken modifiziert werden.

Abhängig von den CAD-Daten erzeugt der Computer 140 anschließend Bearbeitungsdaten, welche angeben, wie ein Knochenersatzstück hergestellt werden soll. Bei der Erzeugung der Bearbeitungsdaten werden auch Parameter wie die Art der Kavität und die Position der Kavität im Körper des Patienten 130 berücksichtigt.

Auf die Bearbeitungsdaten wird anschließend eine Finite-Elemente-Analyse angewendet. Auch dabei werden Daten bezüglich der Position der Kavität im Körper des Patienten 130 sowie Daten bezüglich einer möglichen Okklusion der Kavität und einer umgebenden Knochenstruktur berücksichtigt.

Die erzeugten Bearbeitungsdaten werden anschließend mittels spezieller Algorithmen optimiert, um die nachfolgende automatisierte Bearbeitung und Verwendung der Bearbeitungsdaten so effizient und zuverlässig wie möglich zu gestalten.

Das System 100 weist weiterhin eine Werkzeugmaschine 150 auf. Die im Computer 140 erzeugten und aufbereiteten Bearbeitungsdaten werden an diese Werkzeugmaschine 150 geliefert. Die Werkzeugmaschine 150 weist ein Bearbeitungswerkzeug 155 auf, welches in an sich bekannter Weise in der Werkzeugmaschine 150 aufgenommen wird. Hierbei kann es sich insbesondere um einen Bohrer oder um ein anderes materialabtragendes Gerät handeln.

Benachbart zur Werkzeugmaschine 150 weist das System 100 ferner eine Werkstückhalterung 160 auf. In der Werkstückhalterung 160 ist ein Rohling eines Knochenersatzstücks 10 aufgenommen, um ihn für die Bearbeitung mittels des Werkzeugs 155 zu fixieren. Die Werkzeugmaschine 150 ist dazu ausgebildet, basierend auf den Bearbeitungsdaten vollautomatisiert das Knochenersatzstück 10 zu erzeugen, während es von der Werkstückhalterung 160 gehalten wird.

Nach der Herstellung des Knochenersatzstücks 10 kann dieses separat in den Computertomographen 110 eingeschoben werden, um es zu überprüfen. Hierfür kann beispielsweise eine spezielle Halterung verwendet werden. Dabei werden wiederum entsprechende Daten abhängig von dem vermessenen Knochenersatzstück 10 erzeugt, welche an den Computer 140 geliefert werden. Dieser vergleicht den Ist-Zustand mit dem Soll-Zustand und entscheidet, ob
- das Knochenersatzstück 10 unverändert verwendet werden kann,
- das Knochenersatzstück 10 einer Nachbearbeitung bedarf, oder
- das Knochenersatzstück 10 so schlecht hergestellt wurde, dass es nicht verwendet werden kann und entsorgt werden muss.

Für den Fall einer erforderlichen Nachbearbeitung kann der Computer 140 entsprechende Bearbeitungsdaten für die Werkzeugmaschine 150 erzeugen, welche eine automatisierte Nachbearbeitung des Knochenersatzstücks 10 erlauben. Das Knochenersatzstück 10 kann dann erneut in die Werkstückhalterung 160 zur Nachbearbeitung eingesetzt werden.

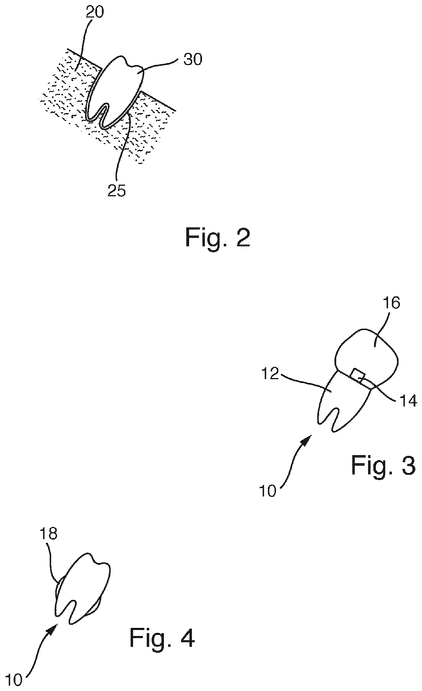

Fig. 2 zeigt einen Abschnitt eines Zahnfleischs 20 mit einer darin ausgebildeten Kavität 25. Es sei verstanden, dass die Struktur des Zahnfleischs 20 durch einen Kieferknochen festgelegt ist, welchen das Zahnfleisch 20 bedeckt. Die Kavität 25 ist also auch in dem Kieferknochen aufgenommen. In die Kavität 25 ist ein Zahn 30 oder Zahnersatz 30 aufgenommen. Es sei erwähnt, dass es sich hierbei insbesondere um ein natürliches Zahnfleisch 20 und einen natürlichen Zahn 30 handeln kann. Die in Fig. 1 gezeigte Vorrichtung 100 kann beispielsweise dazu verwendet werden, um die Kavität 25 und/oder den Zahn oder Zahnersatz 30 zu vermessen und damit einen Ersatz für den Zahn oder Zahnersatz 30 herzustellen, welcher in Form eines Knochenersatzstücks 10 bereitgestellt wird und exakt in die Kavität 25 passt.

Fig. 3 zeigt ein Ausführungsbeispiel eines Knochenersatzstücks 10 in Form eines künstlichen Zahns. Der Zahn ist dabei aufgeteilt in ein Zahnimplantat 12, eine Zahnprothese 16 und eine das Zahnimplantat 12 und die Zahnprothese 16 verbindende Prothesenkronen-Brücken-Absorptionskomponente 14. Diese drei Bestandteile 12, 14, 16 des Zahns 10 können alle separat mittels der Vorrichtung 100 automatisiert hergestellt werden. Wie bereits weiter oben erwähnt kann eine Kavität 25 vermessen werden, wodurch insbesondere die Struktur des Zahnimplantats 12 festgelegt ist. Ebenso kann in dem Computertomographen 110 die Struktur eines Zahns 30 gemessen werden, insbesondere dessen Oberflächenstruktur, um die Struktur der Zahnprothese 16 festzulegen. Die Prothesenkronen-Brücken-Absorptionskomponente 14 kann automatisiert oder auch manuell am Computer 140 erzeugt werden.

Fig. 4 zeigt ein Knochenersatzstück 10, welches als einstückiger Zahn oder Zahnersatz ausgebildet ist. Die mit Bezug auf Fig. 3 erläuterte Trennung in einzelne Bestandteile erfolgt somit nicht. Der Zahn oder Zahnersatz kann vielmehr in einem Arbeitsgang mittels der Werkzeugmaschine 150 aus einem Rohling oder Werkstoff hergestellt werden.

Um die Haftung im Kieferknochen und/oder Zahnfleisch und das Verwachsen mit dem Kieferknochen und/oder Zahnfleisch zu verbessern, ist am Kieferknochen- und/oder Zahnfleischkontaktbereich des Zahns eine Beschichtung 18 aufgebracht. Hierbei handelt es sich um eine poröse Beschichtung, in welche der Kieferknochen und/oder das Zahnfleisch ein- oder anwachsen kann. Eine solche Beschichtung 18 kann insbesondere nach/während der Bearbeitung mittels des Systems 100, beispielsweise in einem chemischen Prozess, aufgebracht werden.

Es sei erwähnt, dass der Computer 140 insbesondere auch Daten bezüglich zu verwendender medizinischer Instrumente sowie Navigationsdaten parallel zu den Bearbeitungsdaten erzeugen kann. Dies erleichtert einem behandelnden Arzt die Bereitstellung der für einen Eingriff notwendigen Instrumente und ermöglicht ihm auch die Verwendung fortgeschrittener Navigations- oder Unterstützungseinrichtungen wie beispielsweise einer Brille oder eines head-up-Displays mit der Möglichkeit, entsprechende Informationen einzublenden. Derartige Ausführungen können auch als Augmented Reality bezeichnet werden.

## Patentansprüche

1. Verfahren zum Herstellen eines Knochenersatzstücks (10), wobei das Knochenersatzstück (10) ein Zahnimplantat (12), ein Abutment und/oder eine Zahnprothese (16) ist oder in Form eines künstlichen Zahns aus einem Zahnimplantat (12), einer Zahnprothese (16) und einer das Zahnimplantat (12) und die Zahnprothese (16) verbindenden Prothesenkronen-Brücken-Absorptionskomponente (14) vorliegt oder als einstückiger Zahnersatz ausgebildet ist,
wobei das Verfahren folgende Schritte aufweist:
- Vermessen einer Kavität (25) in einem Knochen zur Erzeugung von Ausgangsdaten, welche anzeigend sind für eine Struktur der Kavität (25),
- Erzeugen von Bearbeitungsdaten aus den Ausgangsdaten, wobei die Bearbeitungsdaten anzeigend sind für eine Sollstruktur des Knochenersatzstücks (10),
- Herstellen des Knochenersatzstücks (10) durch automatisierte Bearbeitung unter Verwendung der Bearbeitungsdaten, **gekennzeichnet durch** den Schritt
- computerunterstütztes Ermitteln von jeweiligen Typen einer Anzahl von chirurgischen Instrumenten, basierend auf den Ausgangsdaten, den Bearbeitungsdaten, einem Design des Zahnimplantats (12), einem Design des Abutments oder einem Design der Zahnprothese (16).

2. Verfahren nach Anspruch 1,
**dadurch gekennzeichnet, dass**
- die Kavität (25) in einem Unterkiefer oder Oberkiefer zur Aufnahme einer Zahnwurzel angeordnet ist.

3. Verfahren nach Anspruch 1 oder 2,
**dadurch gekennzeichnet, dass**
- der Schritt des Vermessens der Kavität (25) durchgeführt wird, während sich ein Zahn oder Zahnersatz (30) oder eine Zahnwurzel oder ein Zahnwurzelersatz in der Kavität (25) befindet.

4. Verfahren nach einem der vorhergehenden Ansprüche,
**dadurch gekennzeichnet, dass**
- der Schritt des Vermessens der Kavität (25) mittels Computertomographie (CT), Magnetresonanztomographie (MRT) oder Digitaler Volumentomographie (DVT) durchgeführt wird,
und/oder
- die Ausgangsdaten in Computer Aided Design (CAD)-Daten konvertiert werden, und
- basierend auf den CAD-Daten die Bearbeitungsdaten erzeugt werden,
und/oder
- die Bearbeitungsdaten abhängig von der Kavität (25) und/oder einer Position der Kavität (25) erzeugt werden.

5. Verfahren nach einem der vorhergehenden Ansprüche,
**dadurch gekennzeichnet, dass**
- auf die Bearbeitungsdaten vor dem Herstellen des Knochenersatzstücks (10) eine Finite-Elemente-Analyse angewendet wird,
- und zwar insbesondere unter Berücksichtigung von Daten bezüglich Oberkiefer, Unterkiefer, Okklusion und/oder Knochenstruktur.

6. Verfahren nach einem der vorhergehenden Ansprüche,
**gekennzeichnet durch** den Schritt
- Optimieren und/oder Re-designen der Bearbeitungsdaten vor dem Herstellen des Knochenersatzstücks (10).

7. Verfahren nach einem der vorhergehenden Ansprüche,
**gekennzeichnet durch** den Schritt
- Nachbehandeln des Knochenersatzstücks (10) nach dem Herstellen, insbesondere in Abhängigkeit von einer Überprüfung, und insbesondere an einem Wurzelbereich oder an einem Zahnfleischkontaktbereich.

8. Verfahren nach einem der vorhergehenden Ansprüche,
**dadurch gekennzeichnet, dass**
- beim Schritt des Vermessens der Kavität (25) auch ein in der Kavität (25) befindlicher Zahn oder Zahnersatz (30) vermessen wird zur Erzeugung von weiteren Ausgangsdaten, welche anzeigend sind für eine Struktur, insbesondere eine Oberfläche des Zahns (30).

9. Verfahren nach Anspruch 8,
**dadurch gekennzeichnet, dass**
- basierend auf den weiteren Ausgangsdaten weitere Bearbeitungsdaten erzeugt werden, welche anzeigend sind für eine Sollstruktur der Zahnprothese (16) und/oder eine Sollstruktur der Prothesenkronen-Brücken-Absorptionskomponente (14), wobei vorzugsweise
- die weiteren Ausgangsdaten, welche anzeigend sind für die Sollstruktur der Zahnprothese (16), mittels Computer Aided Design (CAD) erzeugt werden.

10. Verfahren nach Anspruch 9,
**gekennzeichnet durch** den Schritt
- Herstellen der Zahnprothese (16) basierend auf den weiteren Ausgangsdaten,
- und zwar insbesondere durch Fräsen und/oder ein generatives Fertigungsverfahren,
- wobei vorzugsweise die Zahnprothese (16) einstückig mit dem Zahnimplantat (12) oder separat vom Zahnimplantat (12) ausgebildet wird.

11. Verfahren nach Anspruch 10,
**gekennzeichnet durch** den Schritt
- Überprüfen der Zahnprothese (16), insbesondere mittels Computertomographie (CT) oder 3D-Scan.

12. Verfahren nach Anspruch 10 oder 11,
**gekennzeichnet durch** den Schritt
- Weiterbehandlung der Zahnprothese (16), insbesondere durch Oberflächenbehandlung,
- wobei die Weiterbehandlung vorzugsweise eine Beschichtung eines Kieferknochen- oder Zahnfleischkontaktbereichs, eine Sterilisation und/oder eine Verpackung beinhaltet.

13. Verfahren nach einem der vorhergehenden Ansprüche,
**gekennzeichnet durch** den Schritt
- Ermitteln von Navigationsinformationen, insbesondere in Bezug auf einen Unterkiefer oder einen Oberkiefer, basierend auf den Ausgangsdaten, den Bearbeitungsdaten, einem Wurzelkanal, einem Design des Implantats (12) und/oder einem Design der Zahnprothese (16).

## Claims

1. A method for producing a bone replacement (10), wherein the bone replacement (10) is a tooth implant 12), an abutment and/or a dental prosthesis (16) or is in the form of an artificial tooth of a tooth implant (12), a dental prosthesis (16) and a prosthesis crown/bridge absorption component (14) that connects the tooth implant (12) and the dental prosthesis (16) or is embodied as an integral tooth replacement, wherein the method includes the following steps:
- measuring a cavity (25) in a bone for producing initial data which are indicative for a structure of the cavity (25),
- producing processing data from the initial data, wherein the processing data are indicative for an intended structure of the bone replacement (10), and
- producing the bone replacement (10) by automated processing using the processing data, **characterized by** the step of
- computer-aided ascertaining respective types of a number of surgical instruments on the basis of the initial data, the processing data, a design of the tooth implant (12), a design of the abutment or a design of the dental prosthesis (16).

2. The method as claimed in claim 1, **characterized in that**
- the cavity (25) is arranged in a mandible or maxilla for receiving a tooth root.

3. The method as claimed in claim 1 or 2, **characterized in that**
- the step of measuring the cavity (25) is carried out while a tooth or tooth replacement (30) or a tooth root or a tooth root replacement is situated in the cavity (25).

4. The method as claimed in any one of the preceding claims,
**characterized in that**
- the step of measuring the cavity (25) is carried out by means of computed tomography (CT), magnetic resonance imaging (MRI) or digital volume tomography (DVT),and/or
- the initial data are converted into computer-aided design (CAD) data and
- the processing data are produced on the basis of the CAD data, and/or
- the processing data are produced dependent on the cavity (25) and/or a position of the cavity (25).

5. The method as claimed in any one of the preceding claims,
**characterized in that**
- a finite element analysis is applied to the processing data before producing the bone replacement (10),
- to be precise, in particular, taking account of data in respect of the maxilla, mandible, occlusion and/or bone structure.

6. The method as claimed in any one of the preceding claims,
**characterized by** the step of
- optimizing and/or redesigning the processing data before producing the bone replacement (10).

7. The method as claimed in any one of the preceding claims,
**characterized by** the step of
- after-treatment of the bone replacement (10) after the production thereof, in particular depending on a check, and in particular at a root region or at a gingiva contact region.

8. The method as claimed in any one of the preceding claims,
**characterized in that**
- a tooth or tooth replacement (30) situated in the cavity (25) is also measured during the step of measuring the cavity (25) for the purposes of producing further initial data which are indicative for a structure, in particular a surface, of the tooth (30).

9. The method as claimed in claim 8, **characterized in that**
- further processing data are produced on the basis of the further initial data, said further processing data being indicative for an intended structure of the dental prosthesis (16) and/or for an intended structure of the prosthesis crown/bridge absorption component (14), wherein preferably
- the further initial data which are indicative for the intended structure of the dental prosthesis (16) are produced by means of computer-aided design (CAD).

10. The method as claimed in claim 9, **characterized by** the step of
- producing the dental prosthesis (16) on the basis of the further initial data,
- to be precise, in particular, by milling and/or a generative manufacturing method,
- wherein preferably the dental prosthesis (16) is embodied in integral fashion with the tooth implant (12) or separately from the tooth implant (12).

11. The method as claimed in claims 10, **characterized by** the step of
- checking the dental prosthesis (16), in particular by means of computed tomography (CT) or a 3D scan.

12. The method as claimed in claim 10 or 11, **characterized by** the step of
- treating the dental prosthesis (16) further, in particular by way of a surface treatment,
- wherein the further treatment preferably includes coating a jawbone or gingiva contact region, sterilizing and/or packaging.

13. The method as claimed in any one of the preceding claims,
**characterized by** the step of
- ascertaining navigation information, in particular in relation to a mandible or a maxilla, on the basis of the initial data, the processing data, a root canal, a design of the implant (12) and/or a design of the dental prosthesis (16).

## Revendications

1. Procédé de fabrication d'une pièce de remplacement osseux (10), la pièce de remplacement osseux (10) étant un implant dentaire (12), un pilier et/ou une prothèse dentaire (16) ou se présentant sous la forme d'une dent artificielle constituée d'un implant dentaire (12), d'une prothèse dentaire (16) et d'un composant d'absorption de couronne prothétique et de pont (14) reliant l'implant dentaire (12) et la prothèse dentaire (16) ou étant réalisée sous la forme d'une prothèse dentaire d'un seul tenant,
le procédé comprenant en outre les étapes suivantes consistant à :
- mesurer une cavité (25) dans un os pour produire des données initiales représentatives d'une structure de la cavité (25),
- produire des données de traitement à partir des données initiales, les données de traitement étant représentatives d'une structure de consigne de la pièce de remplacement osseux (10),
- fabriquer la pièce de remplacement osseux (10) par traitement automatisé en utilisant les données de traitement, **caractérisé par** l'étape consistant à
- déterminer de manière assistée par ordinateur des types respectifs d'un certain nombre d'instruments chirurgicaux, sur la base des données initiales, des données de traitement, d'une conception de l'implant dentaire (12), d'une conception du pilier ou d'une conception de la prothèse dentaire (16).

2. Procédé selon la revendication 1,
**caractérisé en ce que**
- la cavité (25) est disposée dans une mâchoire inférieure ou une mâchoire supérieure pour recevoir une racine dentaire.

3. Procédé selon la revendication 1 ou 2,
**caractérisé en ce que**
- l'étape de mesure de la cavité (25) est effectuée alors qu'une dent ou une prothèse dentaire (30) ou une racine dentaire (25) se trouve dans la cavité.

4. Procédé selon l'une des revendications précédentes, **caractérisé en ce que**
- l'étape de mesure de la cavité (25) est effectuée par tomographie assistée par ordinateur (CT), par imagerie par résonance magnétique (MRT) ou par tomographie volumique numérique (DVT),
et/ou
- les données initiales sont converties en données de conception assistée par ordinateur (CAD), et
- les données de traitement sont générées sur la base des données CAD,
et/ou
- les données de traitement sont générées en fonction de la cavité (25) et/ou d'une position de la cavité (25).

5. Procédé selon l'une des revendications précédentes, **caractérisé en ce que**
- une analyse par éléments finis est appliquée aux données de traitement avant la fabrication de la pièce de remplacement osseux (10),
- cela étant effectué en tenant compte notamment de données relatives à la mâchoire supérieure, à la mâchoire inférieure, à une occlusion et/ou à la structure osseuse.

6. Procédé selon l'une des revendications précédentes, **caractérisé par** l'étape consistant à
- optimiser et/ou reconfigurer les données de traitement avant la fabrication de la pièce de remplacement osseux (10) .

7. Procédé selon l'une des revendications précédentes, **caractérisé par** l'étape consistant à
- soumettre à un post-traitement la pièce de remplacement osseux (10) après sa fabrication, notamment en fonction d'une vérification, et en particulier sur une zone radicalaire ou sur une zone de contact avec la gencive.

8. Procédé selon l'une des revendications précédentes, **caractérisé en ce que**
- lors de l'étape de mesure de la cavité (25), une dent ou une prothèse dentaire (30) se trouvant dans la cavité (25) est également mesurée pour produire d'autres données initiales qui sont représentatives d'une structure, notamment d'une surface de la dent (30).

9. Procédé selon la revendication 8,
**caractérisé en ce que**,
- sur la base des autres données initiales, d'autres données de traitement sont produites, lesquelles sont représentatives d'une structure de consigne de la prothèse dentaire (16) et/ou d'une structure de consigne du composant d'absorption de couronne prothétique et de pont (14), et de préférence,
- les autres données initiales, qui sont représentatives de la structure de consigne de la prothèse dentaire (16), sont produites par conception assistée par ordinateur (CAD) .

10. Procédé selon la revendication 9,
**caractérisé par** l'étape consistant à
- fabriquer la prothèse dentaire (16) sur la base des autres données initiales,
- cela étant notamment effectué par fraisage et/ou par un procédé de fabrication génératif,
- la prothèse dentaire (16) étant de préférence formée d'un seul tenant avec l'implant dentaire (12) ou séparément de l'implant dentaire (12).

11. Procédé selon la revendication 10,
**caractérisé par** l'étape consistant à
- vérifier la prothèse dentaire (16), en particulier par tomographie assistée par ordinateur (CT) ou par scanner 3D.

12. Procédé selon la revendication 10 ou 11, **caractérisé par** l'étape consistant à
- soumettre à un traitement ultérieur la prothèse dentaire (16), notamment par traitement de surface,
- le traitement ultérieur comprenant de préférence le revêtement d'une zone de contact de l'os maxillaire ou de la gencive, une stérilisation et/ou un enveloppement.

13. Procédé selon l'une des revendications précédentes, **caractérisé par** l'étape consistant à
- déterminer des informations de navigation, notamment en ce qui concerne une mâchoire inférieure ou une mâchoire supérieure, sur la base des données initiales, des données de traitement, d'un canal radiculaire, d'une conception de l'implant (12) et/ou d'une conception de la prothèse dentaire (16).
